# EUROPÄISCHE PATENTSCHRIFT

(11) **EP 1 103 180 B1**
(45) Veröffentlichungstag und Bekanntmachung des Hinweises auf die Patenterteilung: **12.01.2005**
(21) Anmeldenummer: 00811083.5
(22) Anmeldetag: 16.11.2000
(51) Int. Cl.: A01N 43/78, C07D 277/24

(54) **Hydroxyphenylvinylthiazole**
Hydroxyphenylvinylthizoles
Hydroxyphenylvinylthiazoles

(30) Priorität: 25.11.1999 EP 99811090
(43) Veröffentlichungstag der Anmeldung: 30.05.2001
(73) Patentinhaber: Ciba Specialty Chemicals Holding Inc., 4057 Basel (CH)
(72) Erfinder: Haap, Wolfgang, 79639 Grenzach-Wyhlen (DE); Hölzl, Werner, 68440 Eschentzwiller (FR); Ochs, Dietmar, 79650 Schopfheim (DE); Puchtler, Karin, 79592 Fischingen (DE); Schnyder, Marcel, 4127 Birsfelden (CH)

(56) Entgegenhaltungen:
- WO-A-94/08982
- US-A- 4 902 700

## Beschreibung

Die vorliegende Erfindung betrifft die Verwendung von Hydroxyphenylvinylthiazolen zur antimikrobiellen Behandlung von Oberflächen, als antimikrobielle Wirksubstanz gegen grampositive und gramnegative Bakterien, Hefen und Pilze sowie Konservierung von Kosmetika, Haushaltsprodukten, Textilien, Kunststoffen und Desinfektionsmitteln.

Die erfindungsgemäss verwendeten Hydroxyphenylvinylthiazole entsprechen der Formel 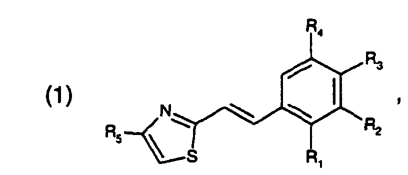 worin
- R₁, R₂, R₃ und R₄,: unabhängig voneinander Wasserstoff; Halogen; Hydroxy, C₁-C₁₆-Alkyl, C₁-C₁₆-Alkoxy, Phenyl; Phenyl-C₁-C₃-Alkyl; C₆-C₁₀-Aryloxy, Amino, Mono-C₁-C₅-Alkylamino, Di-C₁-C₅-Alkylamino, oder -NO₂; wobei mindestens einer der Reste R₁, R₂, R₃ oder R₄ Hydroxy bedeutet;
- R₅: C₁-C₅-Alkyl; C₁-C₅-Alkoxy; nicht substituiertes oder durch Halogen, C₁-C₅-Alkyl, C₁-C₅-Alkoxy, Hydroxy oder Pyrrolidinyl substituiertes Phenyl;
bedeuten.

C₁-C₁₆-Alkyl sind geradkettige oder verzweigte Alkylreste wie z.B. Methyl, Ethyl, n-Propyl, Isopropyl, n-Butyl, sek.Butyl, tert.Butyl, Pentyl, Hexyl, Heptyl, Octyl, Nonyl, Decyl, Undecyl, Dodecyl, Tetradecyl, Pentadecyl oder Hexadecyl.

C₁-C₁₆-Alkoxy bedeutet z.B. Methoxy, Ethoxy, n-Propoxy, Isopropoxy, n-Butoxy, sek.Butoxy, tert.Butoxy oder Pentyloxy, Hexyloxy, Heptyloxy, Octyloxy, Nonyloxy, Decyloxy, Undecyloxy, Dodecyloxy, Tetradecyloxy, Pentadecyloxy oder Hexadecyloxy.

Halogen bedeutet Fluor, Chlor, Brom oder lod.

Die erfindungsgemäss verwendeten Hydroxyphenylvinylthiazole können als E- oder Z-Isomere vorliegen. Vorzugsweise liegen sie als E-Isomere vor.

Interessante Verbindungen sind Hydroxyphenylvinylthiazole der Formel (1), worin R₁, R₂, R₃, R₄, unabhängig voneinander Wasserstoff, Hydroxy, C₁-C₅-Alkyl, C₁-C₅-Alkoxy oder Phenyl-C₁-C₃-Alkyl; bedeuten.

Weiterhin sind Verbindungen der Formel (1) interessant, worin R₅ C₁-C₅-Alkyl; oder nicht substituiertes oder durch Halogen; oder durch Pyrrolidinyl substituiertes Phenyl; bedeuten.

Ganz besonders interessante Verbindungen entsprechen den Formeln 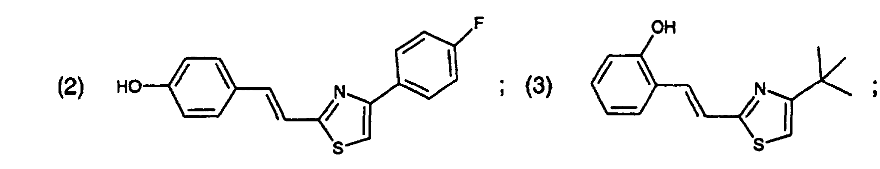 oder

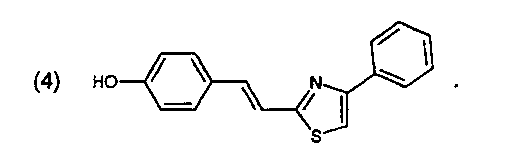

In der folgenden Tabelle 1 sind weitere, beispielhafte, erfindungsgemässe Hydroxyphenylvinylthiazole aufgeführt:

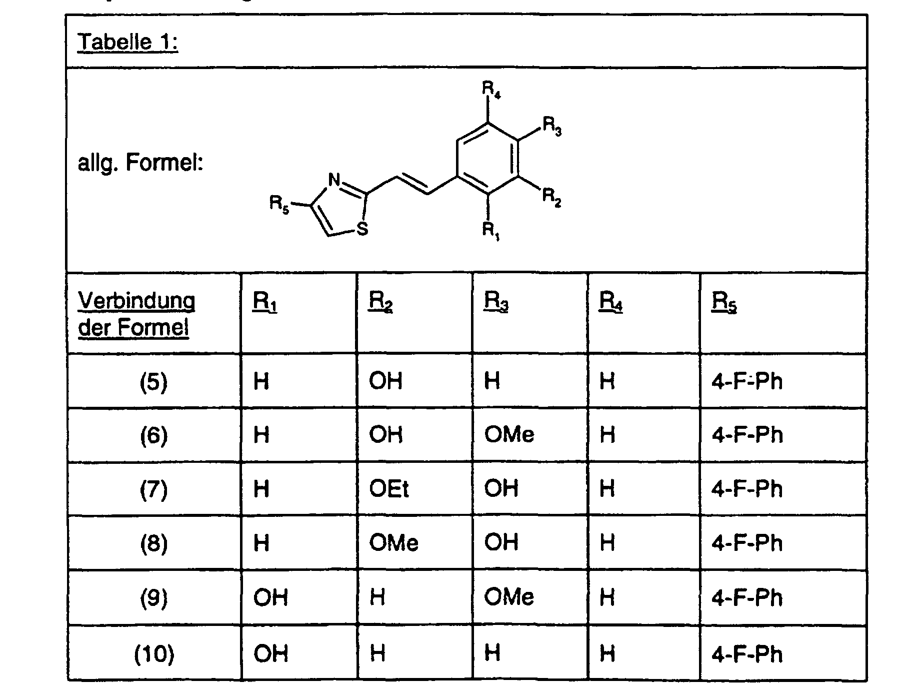

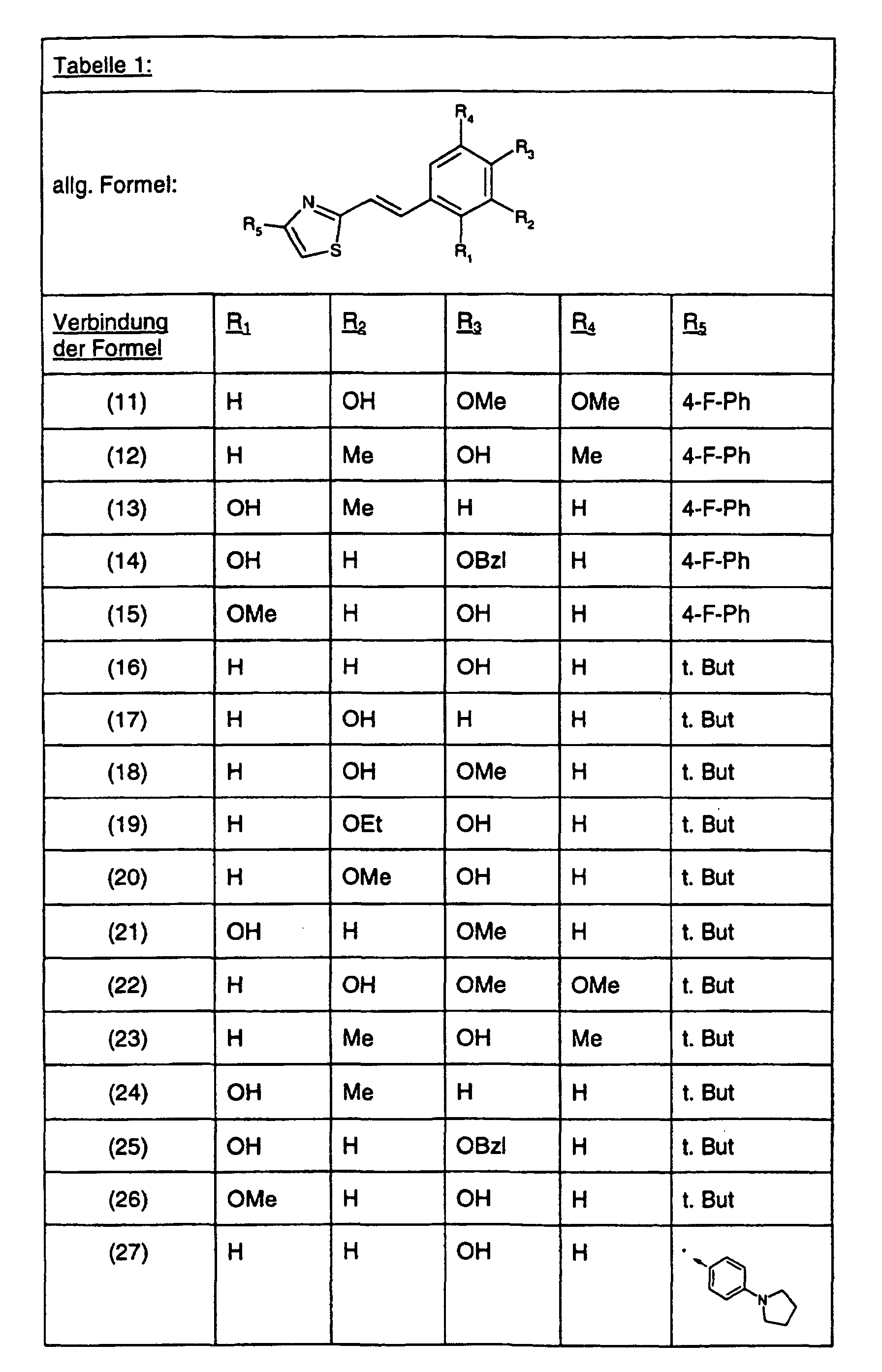

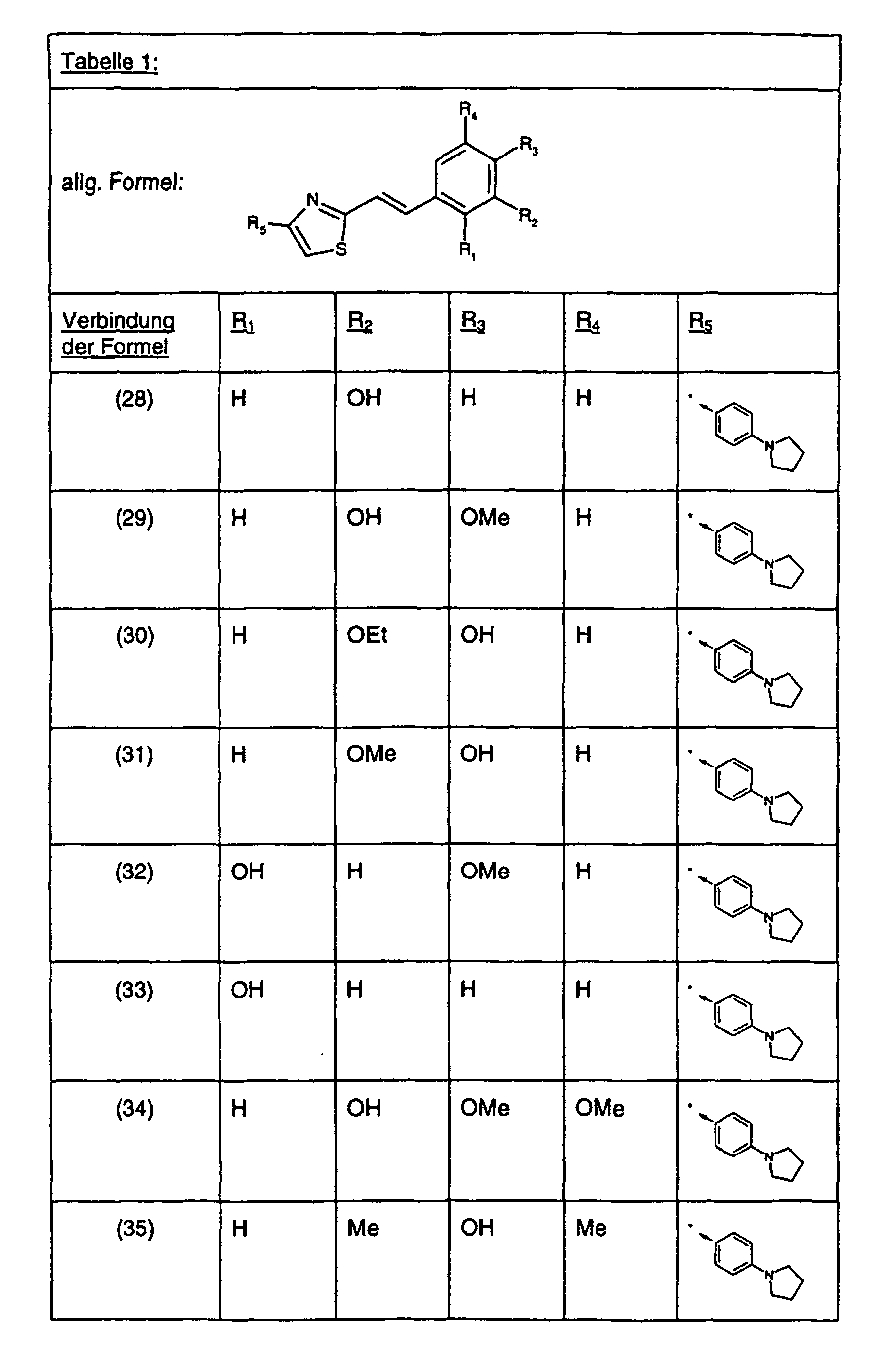

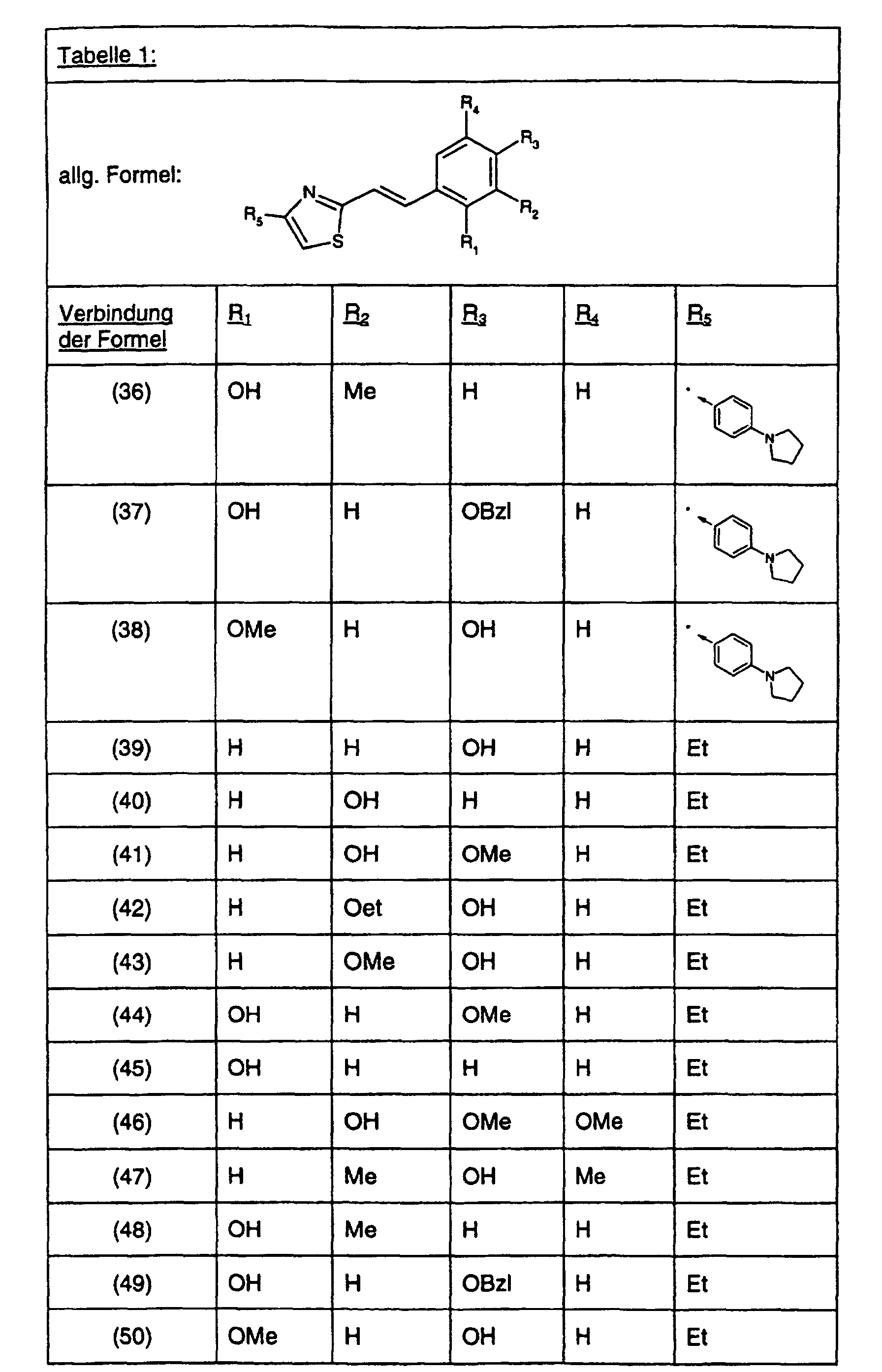

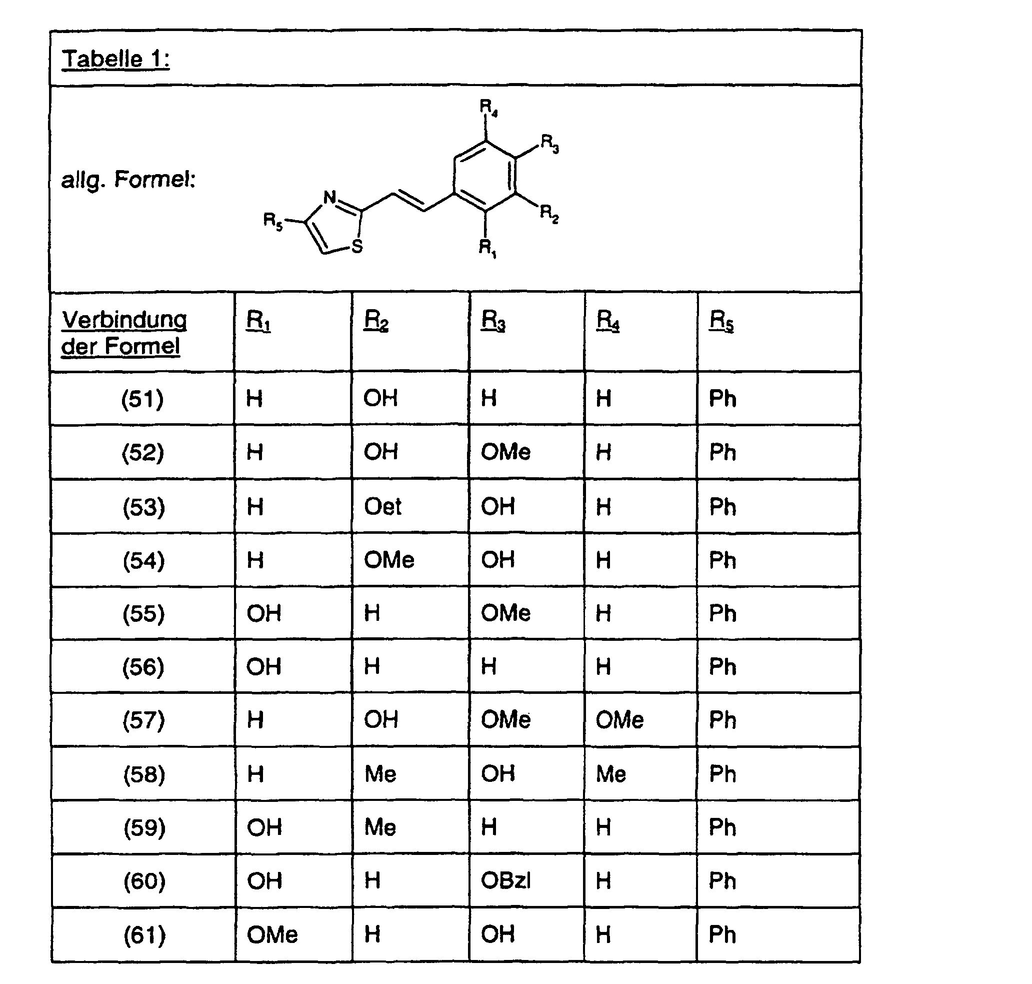

Die Herstellung der erfindungsgemäss verwendeten Hydroxyphenylvinylthiazole erfolgt vorzugsweise in einer Festphasensynthese unter Verwendung eines Tritylharzes. Die Herstellung erfolgt nach folgendem Schema: 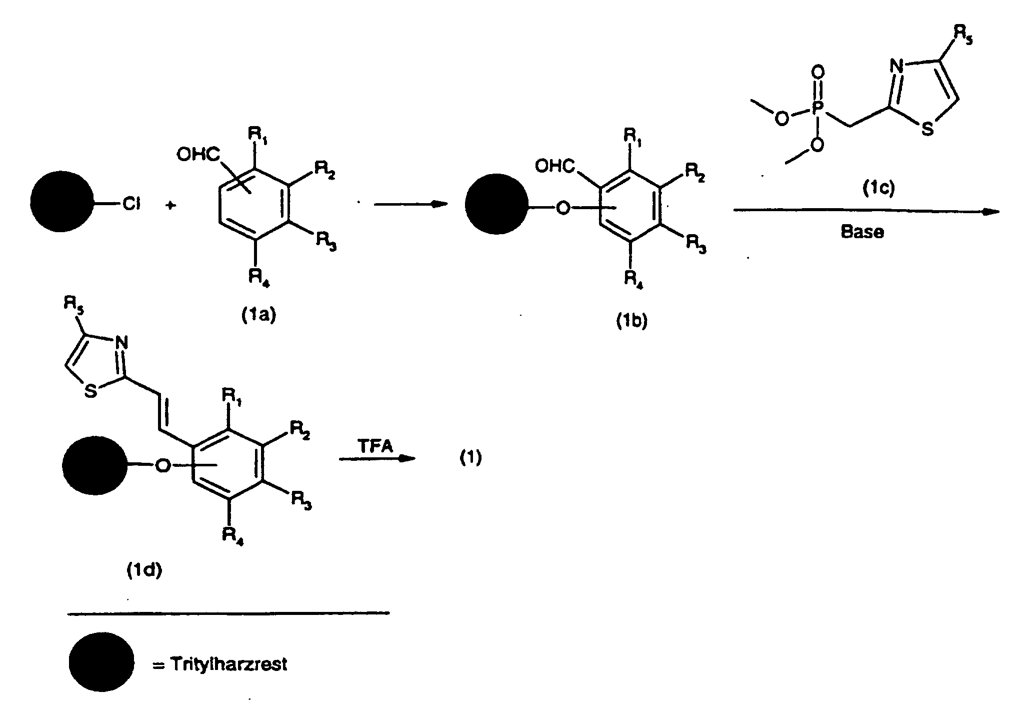 R₁, R₂, R₃, R₄ und R₅ haben dabei die in Formel (1) angegebene Bedeutung.

Die Synthesemethode basiert auf der Literaturvorschrift von R. Willard et al., Chemistry & Biology, *2*, 1995, 45-51. Der Unterschied des erfindungsgemässen Herstellungsverfahrens besteht in der Verwendung des Tritylharzes und der unterschiedlichen Methode zur Beladung des Harzes.

Nähere Einzelheiten über das Herstellungsverfahren sind den entsprechenden Beispielen zu entnehmen.

Die bei der erfindungsgemässen Herstellung als Ausgangsverbindungen eingesetzten Thiazol-2-ylmethylphosphonate (Verbindung der Formel (1c)) erfolgt nach der bekannten Hantzschen Thiazolsynthese:

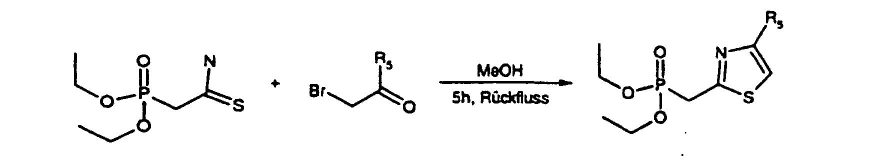

Diethyl(-2-amino-2-thioxoethyl)phosphonat (2,1 g; 10 mmol) und Brommethylketon (10,5 mmol) werden unter Schutzgasatmosphäre in Methanol (10 ml) gelöst. Die Reaktionsmischung wird anschliessend 5 h unter Rückfluss erwärmt und danach zur Trockne eingeengt. Der Rückstand wird in Dichlormethan (100 ml) aufgenommen und mit gesättigter wässriger NaHCO₃-Lösung (2 x 50 ml) und NaCI-Lösung (50 ml) gewaschen. Die Waschlösungen werden mit Dichlormethan (25 ml) extrahiert und die vereinigten organischen Extrakte zur Trockne eingeengt.

Ausbeuten: R₂ = 4-F-Ph (87,1 %); R₂ = t. But (84,7 %); R₂ = 4-(Pyrrolidin-1-yl)-phenyl (98 %); R₂ = Et (86,2 %); R₂ = Ph (100 %).

Sämtliche Verbindungen werden mit ¹H-NMR charakterisiert und zeigen die entsprechenden chemischen Verschiebungen.

Die erfindungsgemäss eingesetzten Hydroxyphenylvinylthiazole zeigen ausgeprägte antimikrobielle Wirkung, insbesondere gegen pathogene grampositive und gramnegative Bakterien sowie gegen Bakterien der Hautflora, ausserdem gegen Hefen und Schimmelpilze. Sie eignen sich daher insbesondere zur Desinfektion, Desodorierung, sowie der allgemeinen und antimikrobiellen Behandlung der Haut und Schleimhäute sowie Hautanhangsgebilde (Haare), ganz besonders zur Hände- und Wunddesinfektion.

Sie sind daher geeignet als antimikrobielle Wirksubstanzen und Konservierungsmittel in Körperpflegemitteln, wie z.B. Shampoos, Badezusätzen, Haarpflegemitteln, flüssigen und festen Seifen (auf Basis synthetischer Tenside und Salze von gesättigten und/oder ungesättigten Fettsäuren), Lotionen und Cremes, Deodorantien, anderen wässrigen oder alkoholischen Lösungen, z.B. Reinigungslösungen für die Haut, feuchten Reinigungstüchern, Ölen oder Pudern.

Einen weiteren Erfindungsgegenstand bildet daher ein Körperpflegemittel, enthaltend mindestens eine Verbindung der Formel (1) sowie kosmetisch verträgliche Träger- oder Hilfsstoffe.

Das erfindungsgemässe Körperpflegemittel enthält 0,01 bis 15, vorzugsweise 0,1 bis 10 Gew.-%, bezogen auf das Gesamtgewicht der Zusammensetzung, der Hydroxyphenylvinylthiazolverbindung der Formel (1) und kosmetisch verträgliche Hilfsstoffe.

Je nachdem, in welcher Form das Körperpflegemittel vorliegt, weist es neben der Hydroxyphenylvinylthiazolverbindung der Formel (1) noch weitere Bestandteile auf, wie z.B. Sequestriermittel, Farbstoffe, Parfümöle, Verdickungs- bzw. Festigungsmittel (Konsistenzregler), Emmollients, UV-Absorber, Hautschutzmittel, Antioxidantien, die mechanischen Eigenschaften verbessernde Additive wie Dicarbonsäuren und/oder Al-, Zn-, Ca-, Mg-Salze von C₁₄-C₂₂-Fettsäuren und gegebenenfalls Konservierungsmittel.

Das erfindungsgemässe Körperpflegemittel kann als Wasser-in-Öl- oder ÖI-in-Was ser-Emulsion, als alkoholische oder alkoholhaltige Formulierung, als vesikulare Dispersion eines ionischen oder nichtionischen amphiphilen Lipids, als Gel, fester Stift oder als AerosolFormulierung formuliert werden.

Als Wasser-in-Öl- oder ÖI-in-Wasser-Emulsion enthält der kosmetisch verträgliche Hilfsstoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikon-Öl, einen Fettsäureester oder einen Fettalkohol. Bevorzugte Mono- oder Polyole sind Ethanol, Isopropanol, Propylenglykol, Hexylenglycol, Glycerin und Sorbitol.

Erfindungsgemässe kosmetische Formulierungen werden in verschiedenen Bereichen eingesetzt. Insbesondere kommen z.B. die folgenden Mittel in Betracht:
- Mittel zur Hautpflege, wie z.B. Hautwasch- und Reinigungsmittel in Form von stückförmigen oder flüssigen Seifen, Syndets oder Waschpasten,
- Badepräparate, wie z.B. flüssige (Schaumbäder, Milche, Duschpräparate) oder feste Badepräparate, wie z.B. Badetabletten und Badesalze;
- Hautpflegemittel, wie z.B. Hautemulsionen, Mehrfachemulsionen oder Hautöle;
- Dekorative Körperpflegemittel, wie z.B. Gesichts-Make-Ups in Form von Tages- oder Pudercremes, Gesichtspuder (lose und gepresst), Rouge oder Creme-Make-Ups, Augenpflegemittel, wie z.B. Lidschattenpräparate, Wimperntusche, Eyeliner, Augencremes oder Eye-Fix-Cremes; Lippenpflegemittel, wie z.B. Lippenstift, Lip Gloss, Lippenkonturstift, Nagelpflegemittel, wie Nagellack, Nagellackentferner, Nagelhärter, oder Nagelhautentferner;
- Intimpflegemittel, wie z.B. Intim-Waschlotionen oder Intimsprays;
- Fusspflegemittel, wie z.B. Fussbäder, Fusspuder, Fusscremes bzw. Fussbalsame, spezielle Deomittel und Antitranspirantien oder hornhautbeseitigende Mittel;
- Lichtschutzmittel, wie Sonnenmilche, -lotionen, -cremes, -öle, Sun-blockers oder Tropicals, Vorbräunungspräparate oder After-sun-Präparate;
- Hautbräunungsmittel, wie z.B. Selbstbräunungscremes;
- Depigmentierungsmittel, wie z.B. Präparate zur Hautbleichung oder Mittel zur Hautaufhellung;
- Insektenabweisende Mittel ("Repellents"), wie z.B. Insektenöle, -lotionen, -sprays, oder - stifte;
- Deodorantien, wie Deosprays, Pumpsprays, Deogele, -stifte oder -roller;
- Antitranspirantien, wie z.B. Antitranspirantstifte, -cremes oder -roller;
- Mittel zur Reinigung und Pflege von unreiner Haut, wie z.B. Syndets (fest oder flüssig), Peeling- oder Scrubb-Präparate oder Peeling-Masken;
- Haarentfernungsmittel in chemischer Form (Depilation), wie z.B. Haarentfernungspulver, flüssige Enthaarungsmittel, cremige oder pastöse Enthaarungsmittel, Enthaarungsmittel in Gelform oder Aerosolschäume;
- Rasiermittel, wie z.B. Rasierseife, schäumende Rasiercremes, nichtschäumende Rasiercremes, -schäume, -gele, Preshave-Präparate für die Trockenrasur, Aftershaves oder Aftershave-Lotionen;
- Duftmittel, wie z.B. Duftwässer (Eau de Cologne, Eau de Toilette, Eau de Parfum, Parfum de Toilette, Parfüm), Parfümöle oder Parfümcremes;
- Mittel zur Zahn-, Zahnersatz- und Mundpflege, wie z.B. Zahncremes, Gel-Zahncremes, Zahnpulver, Mundwasserkonzentrate, Anti-Plaque-Mundspülungen, Prothesenreiniger oder Prothesenhaftmittel;
- Kosmetische Mittel zur Haarbehandlung, wie z.B. Haarwaschmittel in Form von Schampoos, Haarkonditioniermittel, Haarpflegemittel, wie z.B. Vorbehandlungsmittel, Haarwasser, Frisiercremes, Frisiergele, Pomaden, Haarspülungen, Kurpackungen, Intensivhaarkuren, Mittel zur Haarverformung, wie z.B. Wellmittel zur Herstellung von Dauerwellen (Heisswelle, Mildwelle, Kaltwelle), Haarglättungspräparate, flüssige Haarfestiger, Haarschäume, Haarsprays, Blondiermittel, wie. z.B. Wasserstoffperoxidlösungen, aufhellende Schampoos, Blondiercremes, Blondierpulver, Blondierbreie oder -öle, temporäre, semitemporäre oder permanente Haarfärbemittel, Präparate mit selbstoxidierenden Farbstoffen, oder natürliche Haarfärbemittel, wie Henna oder Kamille.

Eine antimikrobielle Seife hat z.B. folgende Zusammensetzung:
0,01 bis 5 Gew.-% der Verbindung der Formel (1)
0,3 bis 1 Gew.-% Titandioxid,
1 bis 10 Gew.-% Stearinsäure
ad 100% Seifengrundlage, wie z.B. die Natriumsalze der Talgfett- und Kokosfettsäure oder Glycerine.

Ein Schampoo hat z.B. die folgende Zusammensetzung:
0,01 bis 5 Gew.-% der Verbindung der Formel (1),
12,0 Gew.-% Natrium-Laureth-2-sulfat,
4,0 Gew.-% Cocamidopropylbetain,
3,0 Gew.-% NaCI und
Wasser ad 100%.

Ein Deodorant hat z.B. die folgende Zusammensetzung:
0,01 bis 5 Gew.-% der Verbindung der Formel (1),
60 Gew.-% Ethanol,
0,3 Gew.-% Parfümöl, und
Wasser ad 100 %.

Einen weiteren Erfindungsgegenstand bildet eine orale Zusammensetzung, enthaltend 0,01 bis 15 Gew.-%, bezogen auf das Gesamtgewicht der Zusammensetzung, der Verbindung der Formel (1) und oral verträgliche Hilfsstoffe.

Beispiel für eine orale Zusammensetzung:
10 Gew.-% Sorbitol,
10 Gew.-% Glycerin,
15 Gew.-% Ethanol,
15 Gew.-% Propylenglykol,
0,5 Gew.-% Natriumlaurylsulfat,
0,25 Gew.-% Natriummethylcocyltaurat,
0,25 Gew.-% Polyoxypropylen/Polyoxyethylen-Blockcopolymer,
0,10 Gew.-% Pfefferminzgeschmacksstoff,
0,1 bis 0,5 Gew.-% einer Verbindung der Formel (1), und
48,6 Gew.-% Wasser.

Die erfindungsgemässe orale Zusammensetzung kann z.B. in Form eines Gels, einer Paste, einer Creme oder einer wässrigen Zubereitung (Mundwasser) vorliegen.

Weiterhin kann die erfindungsgemässe orale Zusammensetzung Verbindungen enthalten, die Fluoridionen freisetzen, die gegen die Bildung von Karies wirksam sind, z.B. anorganische Fluoridsalze, wie z.B. Natrium-, Kalium-, Ammonium- oder Calciumfluorid oder organische Fluoridsalze, wie z.B. Aminfluoride, die unter dem Handelsnamen Olafluor bekannt sind.

Weiterhin eignen sich die erfindungsgemäss verwendeten Hydroxyphenylvinylthiazole der Formel (1) für die Behandlung, insbesondere Konservierung von textilen Fasermaterialien. Es handelt sich dabei um ungefärbte und gefärbte oder bedruckte Fasermaterialien z.B. aus Seide, Wolle, Polyamid oder Polyurethanen, und insbesondere cellulosehaltige Fasermaterialien aller Art. Solche Fasermaterialien sind beispielsweise natürliche Cellulosefasern, wie Baumwolle, Leinen, Jute und Hanf, sowie Zellstoff und regenerierte Cellulose. Bevorzugte geeignete textile Fasermaterialien sind aus Baumwolle.

Die erfindungsgemässen Hydroxyphenylvinylthiazole eignen sich auch zur Behandlung, insbesondere zur antimikrobiellen Ausrüstung oder Konservierung von Kunststoffen, wie z.B. Polyethylen, Polypropylen, Polyurethan, Polyester, Polyamid, Polycarbonat, Latex etc.. Einsatzbereiche dafür sind z.B. Fussbodenbeläge, Kunststoffbeschichtungen, Kunststoffbehälter- und Verpackungsmaterialien; Küchen- und Badezimmer-Utensilien (z.B. Bürsten, Duschvorhänge; Schwämme, Badezimmermatten), Latex, Filtermaterialien (Luft- und Wasserfilter), Kunststoffartikel, die im medizinischen Bereich eingesetzt werden, wie z.B. Verbandmaterialien, Spritzen, Katheter etc., sog. "medical devices", Handschuhe und Matratzen.

Auch Papier, wie z.B. Hygienepapiere können mit den erfindungsgemässen Hydroxyphenylvinylthiazolen antimikrobiell ausgerüstet werden.

Weiterhin können Nonwovens, wie z.B. Windeln, Damenbinden, Dameneinlagen, Tücher für den Hygiene- und Haushaltsbereich erfindungsgemäss antimikrobiell ausgerüstet werden.

Weiterhin finden die Hydroxyphenylvinylthiazole der Formel (1) Verwendung in Wasch- und Reinigungsformulierungen, wie z.B. in Flüssig- und Pulverwaschmitteln oder Weichspülern.

Die Hydroxyphenylvinylthiazole können insbesondere auch in Haushalts- und Allzweckreinigern zur Reinigung und Desinfektion von harten Oberflächen eingesetzt werden.

Ein Reinigungsmittel hat z.B. folgende Zusammensetzung:

| | |
|---|---|
| 0,01 bis 5 % | der Verbindung der Formel (1) |
| 3,0 % | Octylalkohol 4EO |
| 1,3 % | Fettalkohol C₈-C₁₀-Polyglucosid |
| 3,0 % | Isopropanol |
| ad 100 % | Wasser. |

Neben der Konservierung von Kosmetik- und Haushaltsprodukten ist auch die Konservierung und antimikrobielle Ausrüstung von technischen Produkten sowie der Einsatz als Biozid in technischen Prozessen möglich, wie z.B. bei der Papierbehandlung, insbesondere in Papierbehandlungsflotten, Druckverdickern aus Stärke oder Celluloseabkömmlingen, Lacken und Anstrichfarben.

Die Hydroxyphenylvinylthiazolverbindungen der Formel (1) eignen sich auch zur antimikrobiellen Holzbehandlung sowie zur antimikrobiellen Behandlung, Konservierung und Ausrüstung von Leder.

Weiterhin eignen sich die erfindungsgemässen Verbindungen zum Schutz von kosmetischen Produkten und Haushaltsprodukten vor mikrobieller Verderbnis.

Bei den erfindungsgemäss verwendbaren Hydroxyphenylvinylthiazolverbindungen handelt es sich um bekannte Verbindungen.

Die folgenden, die Erfindung nicht einschränkenden Beispiele dienen der Veranschaulichung.

### Allgemeine Synthesevorschrift der neuen Verbindungen

a) Tritylchlorid-Harz (100 mg, 0,1 mmol) wird in Dichlormethan (2 ml) gequollen und mit einem der weiter unten angegebenen Hydroxybenzaldehyden (10 eq, 1 mmol), DIPEA (10 eq, 1 mmol) sowie einer katalytischen Menge DMAP versetzt. Die Mischung wird 3 h bei 25 °C geschüttelt. Das Harz wird danach abgesaugt und intensiv mit DMF, Methanol, Dichlormethan und Methanol gewaschen und im Vakuum getrocknet.
   IR: 1690 cm⁻¹ (C=O Valenzschwingung des Aldehyds)
b) Das belegte Harz (100 mg, 0,1 mmol) wird anschliessend in DMF (abs., 3 mL) suspendiert und mit einem der u.a. 2-Thiazolylmethylphosphonaten (10 eq, 1 mmol) sowie Natriumethanolat bzw. Natriummethanolat (10 eq, 1 mmol) versetzt. Die Mischung wird 18 h bei 25°C geschüttelt. Danach wird das Harz abgesaugt und intensiv wie oben gewaschen und getrocknet.
   IR: C=O Valenzschwingung ist komplett verschwunden
c) Zur Abspaltung wird das Harz mit 5% TFA in Dichlormethan (3 ml) versetzt und 3 h bei 25°C geschüttelt. Die Abspaltlösung wird abfiltriert und zur Trockne eingeengt. Der Rückstand wird aus tert. Butylalkohol/Wasser (4:1) gefriergetrocknet.

Sämtliche Verbindungen werden mit LC-MS analysiert und zeigen die erwarteten Massen. Die Reinheiten der Verbindungen betragen zwischen 40 - 98 % (Peakflächen im Chromatogramm; UV-Detektion bei 254 nm).

Einige der Verbindungen werden mit ¹H-NMR näher charakterisiert. Die Verbindungen liegen demnach hauptsächlich in der E-Konfiguration vor.

Bspiel: Verbindung der Formel (2):
LC-MS: Z-lsomer [M+H]⁺ 298; 1,7 %
*E*-Isomer [M+H]⁺ 298; 96,9 %
Detektion bei 254 nm
¹H-NMR DMSO-D6 [ppm; Hz]: 9,95 (s, breit, OH); 8,10 (dd 8,58 Hz, Kopplung mit F), 8,07 (s, Thiazol-Proton), 7,62 (d, 8,58 Hz), 7,51 (d, 15,8 Hz Vinyl-Proton), 7,38 (d, 15,8 Hz Vinyl-Proton), 7,36 (d, 8,58 Hz), 6,87 (d, 8,58 Hz).

### Verwendete Bausteine:

Hydroxybenzaldehyde OHC-R₁:
R₁ =

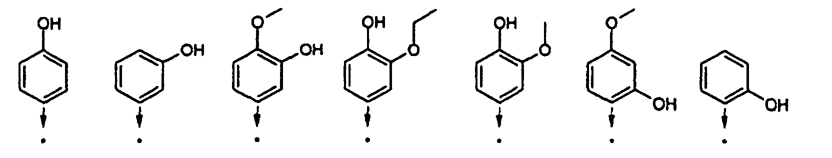

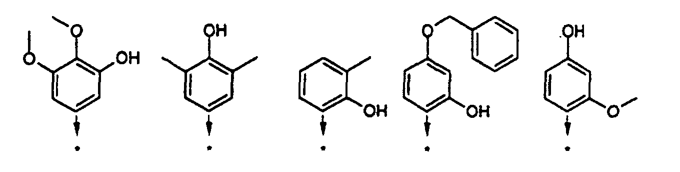

Phosphonate: 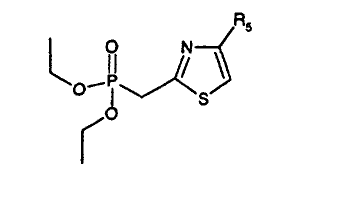 R₅ =

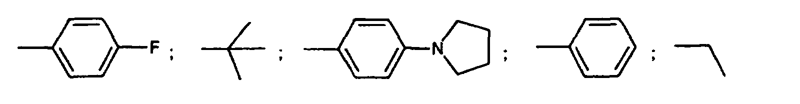

Mit dieser Methode wird eine Matrix von 12 x 5 = 60 Hydroxyphenylvinylthiazolen erhalten. Die Verbindungen sind in Tabelle 1 aufgeführt.

Die ermittelten mikrobiologischen Daten sind in Tabelle 2 zusammengefasst.

**Tabelle 2:**

| MHK-Werte in ppm bei verschiedenen Mikroorganismen * ) | | | | | |
|---|---|---|---|---|---|
| Verbindung d. F. | S. aureus | E. coli | P. aeruginosa | C. albicans | A. niger |
| (2) | >100 | >100 | >100 | 30 | >100 |
| (3) | 60 | >100 | >100 | >100 | >100 |
| (4) | >100 | >100 | >100 | 30 | >100 |
| (5) | >100 | >100 | >100 | >100 | >100 |
| (6) | >100 | >100 | >100 | >100 | >100 |
| (7) | >100 | >100 | >100 | >100 | >100 |
| (8) | >100 | >100 | >100 | >100 | >100 |
| (9) | >100 | >100 | >100 | >100 | >100 |
| (10) | >100 | >100 | >100 | >100 | >100 |
| (11) | >100 | >100 | >100 | >100 | >100 |
| (12) | >100 | >100 | >100 | >100 | >100 |
| (13) | >100 | >100 | >100 | >100 | >100 |
| (14) | >100 | >100 | >100 | >100 | >100 |
| (15) | >100 | >100 | >100 | >100 | >100 |
| (16) | >100 | >100 | >100 | >100 | >100 |
| (17) | >100 | >100 | >100 | >100 | >100 |
| (18) | >100 | >100 | >100 | >100 | >100 |
| (19) | >100 | >100 | >100 | >100 | >100 |
| (20) | >100 | >100 | >100 | >100 | >100 |
| (21) | >100 | >100 | >100 | >100 | >100 |
| (22) | >100 | >100 | >100 | >100 | >100 |
| (23) | >100 | >100 | >100 | >100 | >100 |
| (24) | >100 | >100 | >100 | >100 | >100 |
| (25) | >100 | >100 | >100 | >100 | >100 |
| (26) | >100 | >100 | >100 | >100 | >100 |
| (27) | >100 | >100 | >100 | >100 | >100 |
| (28) | >100 | >100 | >100 | >100 | >100 |
| (29) | >100 | >100 | >100 | >100 | >100 |
| (30) | >100 | >100 | >100 | >100 | >100 |
| (31) | >100 | >100 | >100 | >100 | >100 |
| (32) | >100 | >100 | >100 | >100 | >100 |
| (33) | >100 | >100 | >100 | >100 | >100 |
| (34) | >100 | >100 | >100 | >100 | >100 |
| (35) | >100 | >100 | >100 | >100 | >100 |
| (36) | >100 | >100 | >100 | >100 | >100 |
| (37) | >100 | >100 | >100 | >100 | >100 |
| (38) | >100 | >100 | >100 | >100 | >100 |
| (39) | >100 | >100 | >100 | >100 | >100 |
| (40) | >100 | >100 | >100 | >100 | >100 |
| (41) | >100 | >100 | >100 | >100 | >100 |
| (42) | >100 | >100 | >100 | >100 | >100 |
| (43) | >100 | >100 | >100 | >100 | >100 |
| (44) | >100 | >100 | >100 | >100 | >100 |
| (45) | >100 | >100 | >100 | >100 | >100 |
| (46) | >100 | >100 | >100 | >100 | >100 |
| (47) | >100 | >100 | >100 | >100 | >100 |
| (48) | >100 | >100 | >100 | >100 | >100 |
| (49) | >100 | >100 | >100 | >100 | >100 |
| (50) | >100 | >100 | >100 | >100 | >100 |
| (51) | >100 | >100 | >100 | >100 | >100 |
| (52) | >100 | >100 | >100 | >100 | >100 |
| (53) | >100 | >100 | >100 | >100 | >100 |
| (54) | >100 | >100 | >100 | >100 | >100 |
| (55) | >100 | >100 | >100 | >100 | >100 |
| (56) | >100 | >100 | >100 | >100 | >100 |
| (57) | >100 | >100 | >100 | >100 | >100 |
| (58) | >100 | >100 | >100 | >100 | >100 |
| (59) | >100 | >100 | >100 | >100 | >100 |
| (60) | >100 | >100 | >100 | >100 | >100 |
| (61) | >100 | >100 | >100 | >100 | >100 |

| | | | | | |
|---|---|---|---|---|---|
| *) Die MHK-Werte wurden über Messung der optische Dichte bei Konzentrationen der Substanzen zwischen 100; 10 und 1 ppm ermittelt. Insofern sind einige der Daten Richtwerte der Aktivität. Die MHK-Werte der Verbindungen mit guter Aktivität (Verbindungen der Formeln (2), (3) und (4)) wurden über Messung der optischen Dichte bei Konzentrationen zwischen 120; 60; 30; 15; 7,5; 3,75 ppm ermittelt. | | | | | |

### Bestimmung der minimalen Hemmkonzentration (MHK-Wert) in Mikrotiterplatten: Nährmedium:

Casein-Sojamehl-Pepton-Bouillon zur Herstellung der Vorkulturen der Testbakterien und Hefe.
Mycological Schrägagar zur Vorkultur von Schimmelpilzen

| Beispiele für Testkeime: | |
|---|---|
| Bakterien | Staphylococcus hominis DMS 20328 |
| | Escherichia coli NCTC 8196 |
| | Pseudomonas aeruginosa CIP A-22 |
| | |
| Hefe | Candida albicans ATCC 10231 |
| | |
| Schimmelpilz | Aspergillus niger ATCC 6275 |

### Durchführung:

Die Testsubstanzen werden in Dimethylsulfoxid (DMSO) vorgelöst und in einer Verdünnungsreihe von 1:2 getestet.

Bakterien und Hefe werden über Nacht in CASO-Bouillon, der Schimmelpilz auf Mycological-Schrägagar angezüchtet und mit 10 ml 0,85 % Kochsalzlösung (+ 0,1% TritonX-100) abgeschwämmt.

Alle Testkeime werden mit 0,85 %iger Kochsalzlösung auf eine Keimzahl von 1 - 5 x 10⁶ KBE/ml eingestellt.

Die Testsubstanzen werden à 8 µl pro well in Mikrotiterplatten vorpipettiert.

Vorverdünnte Keimsuspensionen werden 1:100 in CASO-Bouillon (Bakterien und Hefe) bzw. Sabouraud 2% Glucsoe-Bouillon (Schimmelpilz) verdünnt und ä 192 µl pro well den Testsubstanzen zugegeben.

Die Testansätze werden 48 Stunden bei 37°C (Bakterien und Hefe) bzw. 5 Tage bei 28°C (Schimmelpilz) inkubiert.

Nach Inkubation wird das Wachstum anhand der Trübung der Testansätze (optische Dichte) bei 620 nm in einem Mikroplate-Reader bestimmt.

Als minimale Hemmkonzentration (MHK-Wert) wird diejenige Substanzkonzentration angegeben, bei der (verglichen mit der Wachstumskontrolle) eine deutliche Wachstumshemmung (≤ 20 % Wachstum) der Testkeime festzustellen ist.

Pro Testkeim und Substanzkonzentration wird eine Mikrotiterplatte angesetzt. Alle Substanzen werden im Doppel geprüft.

## Patentansprüche

1. Nichttherapeutische Verwendung von Hydroxyphenylvinylthiazolen der Formel 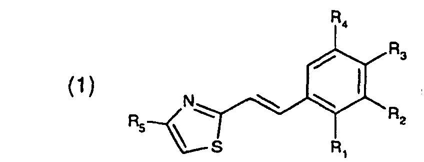 worin
R₁, R₂, R₃ und R₄, unabhängig voneinander Wasserstoff; Halogen; Hydroxy, C₁-C₁₆-Alkyl, C₁-C₁₆-Alkoxy, Phenyl; Phenyl-C₁-C₃-Alkyl; C₆-C₁₀-Aryloxy, Amino, Mono-C₁-C₅-Alkylamino, Di-C₁-C₅-Alkylamino, oder -NO₂; wobei mindestens einer der Reste R₁, R₂, R₃ oder R₄ Hydroxy bedeutet;
R₅ C₁-C₅-Alkyl; C₁-C₅-Alkoxy; nicht substituiertes oder durch Halogen, C₁-C₅-Alkyl, C₁-C₅-Alkoxy, Hydroxy oder Pyrrolidinyl substituiertes Phenyl;
bedeuten, zur antimikrobiellen Behandlung von Oberflächen.

2. Verwendung nach Anspruch 1, **dadurch gekennzeichnet, dass**
R₁, R₂, R₃, R₄, unabhängig voneinander Wasserstoff, Hydroxy, C₁-C₅-Alkyl, C₁-C₅-Alkoxy oder Phenyl-C₁-C₃-Alkyl; bedeuten.

3. Verwendung nach Anspruch 1 oder 2, **dadurch gekennzeichnet, dass**
R₅ C₁-C₅-Alkyl; oder nicht substituiertes oder durch Halogen; oder durch Pyrrolidinyl substituiertes Phenyl; bedeuten.

4. Verwendung nach einem der Ansprüche 1 bis 3, **dadurch gekennzeichnet, dass** die Verbindung der Formel 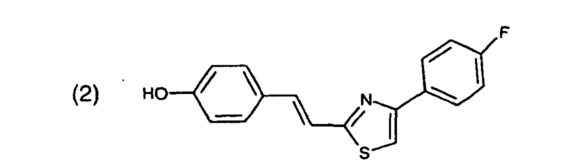 verwendet wird.

5. Verwendung nach einem der Ansprüche 1 bis 3, **dadurch gekennzeichnet, dass** die Verbindung der Formel 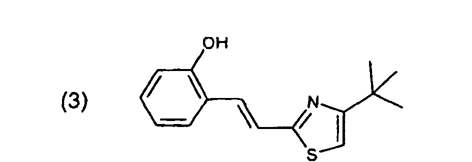 verwendet wird.

6. Verwendung nach einem der Ansprüche 1 bis 3, **dadurch gekennzeichnet, dass** die Verbindung der Formel 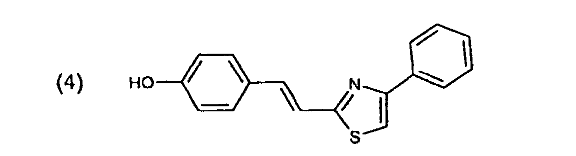 verwendet wird.

7. Verwendung nach einem der Ansprüche 1 bis 6, **dadurch gekennzeichnet, dass** die Verbindungen der Formel (1) in der E- oder Z-Form vorliegen.

8. Verwendung nach Anspruch 7, **dadurch gekennzeichnet, dass** die Verbindungen der Formel (1) in der E-Form vorliegen.

9. Nichttherapeutische Verwendung der Verbindung der Formel (1) zur antimikrobiellen Behandlung, Desodorierung und Desinfektion der Haut, Schleimhäute und Haare.

10. Verwendung nach Anspruch 9, **dadurch gekennzeichnet, dass** die Verbindung der Formel (1) zur Desinfektion und Desodorierung verwendet wird.

11. Verwendung der Verbindung der Formel (1) zur Behandlung von textilen Fasermaterialien.

12. Verwendung nach Anspruch 11, **dadurch gekennzeichnet, dass** die Verbindung der Formel (1) zur Konservierung verwendet wird.

13. Verwendung der Verbindung der Formel (1) in Wasch- und Reinigungsformulierungen.

14. Verwendung der Verbindung der Formel (1) zur antimikrobiellen Ausrüstung und Konservierung von Kunststoffen, Papier, Nonwovens, Holz oder Leder.

15. Verwendung der Verbindung der Formel (1) zur antimikrobiellen Ausrüstung und Konservierung von technischen Produkten, insbesondere Druckverdickern aus Stärke oder Celluloseabkömmlingen, Lacken und Anstrichfarben.

16. Verwendung der Verbindung der Formel (1) als Biozid in technischen Prozessen.

17. Körperpflegemittel, enthaltend
0,01 bis 15 Gew.-%, bezogen auf das Gesamtgewicht der Zusammensetzung, der Verbindung der Formel (1) und kosmetisch verträgliche Hilfsstoffe.

18. Orale Zusammensetzung, enthaltend 0,01 bis 15 Gew.-%, bezogen auf das Gesamtgewicht der Zusammensetzung, der Verbindung der Formel (1) und oral verträgliche Hilfsstoffe.

## Claims

1. The non-therapeutic use of a hydroxyphenylvinylthiazole of formula 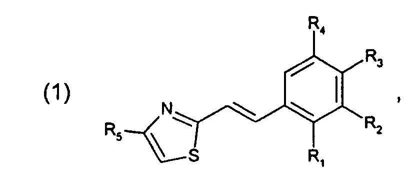 wherein
R₁, R₂, R₃ and R₄ are each independently of the others hydrogen; halogen; hydroxy, C₁-C₁₆-alkyl, C₁-C₁₆alkoxy, phenyl; phenyl-C₁-C₃alkyl; C₆-C₁₀aryloxy, amino, mono-C₁-C₅alkylamino, di-C₁-C₅alkylamino or -NO₂; wherein at least one of the radicals R₁, R₂, R₃ and R₄ is hydroxy;
R₅ is C₁-C₅alkyl; C₁-C₅alkoxy; or unsubstituted or halo-, C₁-C₅alkyl-, C₁-C₅alkoxy-, hydroxy- or pyrrolidinyl-substituted phenyl;
in the antimicrobial treatment of surfaces.

2. Use according to claim 1, wherein
R₁, R₂, R₃ and R₄ are each independently of the others hydrogen, hydroxy, C₁-C₅alkyl, C₁-C₅-alkoxy or phenyl-C₁-C₃alkyl.

3. Use according to either claim 1 or claim 2, wherein
R₅ is C₁-C₅alkyl; or unsubstituted or halo- or pyrrolidinyl-substituted phenyl.

4. Use according to any one of claims 1 to 3, wherein the compound of formula 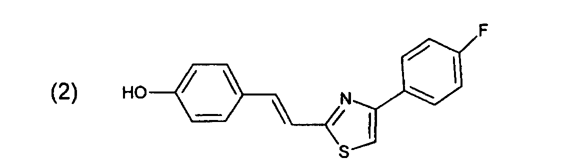 is used.

5. Use according to any one of claims 1 to 3, wherein the compound of formula 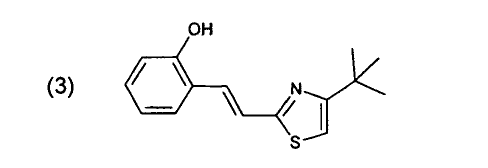 is used.

6. Use according to any one of claims 1 to 3, wherein the compound of formula 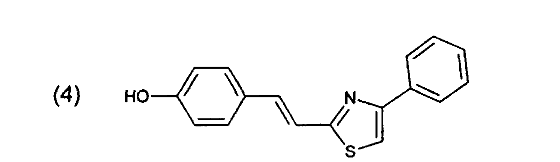 is used.

7. Use according to any one of claims 1 to 6, wherein the compound of formula (1) is in the E or Z form.

8. Use according to claim 7, wherein the compound of formula (1) is in the E form.

9. The non-therapeutic use of a compound of formula (1) in the antimicrobial treatment, deodorisation and disinfection of the skin, mucosa and hair.

10. Use according to claim 9, wherein the compound of formula (1) is used for disinfection and deodorisation.

11. The use of a compound of formula (1) in the treatment of textile fibre materials.

12. Use according to claim 11, wherein the compound of formula (1) is used for preservation.

13. The use of a compound of formula (1) in washing and cleaning formulations.

14. The use of a compound of formula (1) in the antimicrobial dressing and preservation of plastics, paper, nonwovens, wood or leather.

15. The use of a compound of formula (1) in the antimicrobial dressing and preservation of industrial products, especially printing thickeners consisting of starch or cellulose derivatives, surface coatings and paints.

16. The use of a compound of formula (1) as a biocide in industrial processes.

17. A personal care preparation comprising
from 0.01 to 15 % by weight, based on the total weight of the composition, of a compound of formula (1), and cosmetically tolerable adjuvants.

18. An oral composition comprising from 0.01 to 15 % by weight, based on the total weight of the composition, of a compound of formula (1), and orally tolerable adjuvants.

## Revendications

1. Utilisation non thérapeutique d'hydroxyphénylvinyl-thiazoles de formule 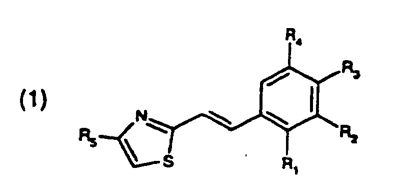 dans laquelle R₁, R₂, R₃ et R₄, chacun indépendamment des autres, représentent l'hydrogène ; un halogène ; le groupe hydroxy, un groupe alkyle en C₁ à C₁₆, un groupe alcoxy en C₁ à C₁₆, un groupe phényle ; un groupe phényl-alkyle en C₁ à C₃ ; un groupe aryloxy en C₆ à C₁₀, un groupe amino, un groupe mono-C₁-C₅-alkylamino, un groupe di-C₁-C₅-alkylamino, ou -NO₂ ; dans laquelle au moins un des restes R₁, R₂, R₃ ou R₄ représente un hydroxy ;
R₅ représente un groupe alkyle en C₁ à C₅ ; un groupe alcoxy en C₁ à C₅ ; un groupe phényle non substitué ou substitué par un halogène, un groupe alkyle en C₁ à C₅, un groupe alcoxy en C₁ à C₅, un groupe hydroxy ou pyrrolidinyle, pour le traitement antimicrobien des surfaces.

2. Utilisation selon la revendication 1, caractérisée ce que R₁, R₂, R₃ et R₄ représentent chacun indépendamment des autres l'hydrogène, le groupe hydroxy, un groupe alkyle en C₁ à C₅, un groupe alcoxy en C₁ à C₅, ou phényl-alkyle en C₁ à C₃.

3. Utilisation selon la revendication 1 ou la revendication 2, **caractérisée en ce que** R₅ représente un groupe alkyle en C₁ à C₅ ; ou bien un groupe phényle non substitué ou substitué par un halogène ; ou par un groupe pyrrolidinyle.

4. Utilisation selon l'une quelconque des revendications 1 à 3, **caractérisée en ce que** le composé de formule 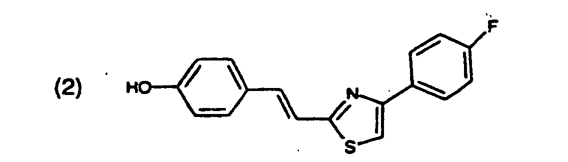 est utilisé.

5. Utilisation selon l'une quelconque des revendications 1 à 3, **caractérisée en ce que** le composé de formule 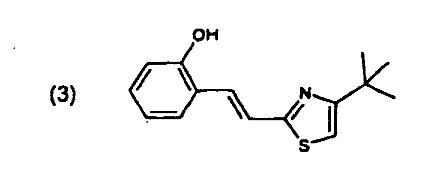 est utilisé.

6. Utilisation selon l'une quelconque des revendications 1 à 3, **caractérisée en ce que** le composé de formule 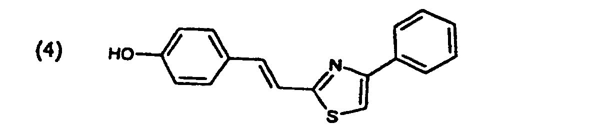 est utilisé.

7. Utilisation selon l'une quelconque des revendications 1 à 6, **caractérisée en ce que** le composé de formule (1) est sous la forme E ou la forme Z.

8. Utilisation selon la revendication 7, **caractérisée en ce que** le composé de formule (1) est sous la forme E.

9. Utilisation non thérapeutique du composé de formule (1) pour un traitement antimicrobien, désodorisant et désinfectant de la peau, des muqueuses et des cheveux.

10. Utilisation selon la revendication 9, **caractérisée en ce que** le composé de formule (1) est utilisé pour la désinfection et la désodorisation.

11. Utilisation du composé de formule (1) pour le traitement de matériaux en fibres textiles.

12. Utilisation selon la revendication 11, **caractérisée en ce que** le composé de formule (1) est utilisé pour la conservation.

13. Utilisation du composé de formule (1) dans les formulations de lavage et de nettoyage.

14. Utilisation du composé de formule (1) pour l'apprêt antimicrobien et la conservation des plastiques, du papier, des non-tissés, du bois et du cuir.

15. Utilisation du composé de formule (1) pour l'apprêt antimicrobien et la conservation des produits techniques, en particulier les agents épaississants pour l'imprimerie à base d'amidon ou de dérivés de la cellulose, les vernis et les peintures.

16. Utilisation du composé de formule (1) en tant que biocide dans les procédés techniques.

17. Produit de soin corporel contenant 0,01 à 15 % en poids, par rapport au poids total de la composition, du composé de formule (1) et des additifs tolérés en cosmétologie.

18. Composition orale, contenant 0,01 à 15 % en poids, par rapport au poids total de la composition, du composé de formule (1) et des additifs tolérés par voie orale.
